# EUROPEAN PATENT SPECIFICATION

(11) **EP 3 544 581 B1**
(45) Date of publication and mention of the grant of the patent: **08.11.2023**
(21) Application number: 16801245.8
(22) Date of filing: 25.11.2016
(51) Int. Cl.: A61K 8/73, A61K 8/34, A61K 8/99, A61K 8/02, A61K 8/81, A61K 8/46, A61Q 19/00

(54) **AQUEOUS COSMETIC**
WÄSSRIGES KOSMETIKUM
PRODUIT COSMÉTIQUE AQUEUX

(43) Date of publication of application: 02.10.2019
(73) Proprietor: LvmH Recherche, 45800 Saint-Jean De Braye (FR)
(72) Inventor: HATA, Koichi, Tokyo 102-0092 (JP); OZAWA, Mai, Tokyo 102-0092 (JP); SAKODA, Takayoshi, Tokyo 102-0092 (JP)
(74) Representative: Cabinet Beau de Loménie
(86) International application number: PCT/EP2016/078777
(87) International publication number: WO 2018/095532

(56) References cited:
- JP-A- S61 289 011
- US-A1- 2007 066 499
- US-A1- 2009 247 444
- US-A1- 2009 280 149
- US-A1- 2016 129 119

## Description

### Technical Field

The present invention relates to an aqueous cosmetic.

### Background Art

For the purpose of preventing dryness of skin after face washing, conventional lotions having moisture-retaining property are used.

Such lotions are in the form of liquid cosmetics that are generally composed of a moisturizing agent and water. Some of them are transparent and others are made opalescent in order to impart luxurious sensation.

### Technical Problem

Opalescence lotions have already been proposed. One of them is a microemulsion with relatively high viscosity where oil droplets are dispersed in an aqueous phase. This type of lotion imparts richness sensation and nourishing effect; however, it has a high viscosity because of a large amount of a thickener contained therein. It also fails to deliver watery fresh sensation upon contact with the skin.

Japanese Unexamined Patent Publication No. 2000-95631 describes such an opalescent lotion type that cosmetic contains (a) water, (b) one or more kinds selected from an anionic surfactant, a cationic surfactant, a nonionic surfactant, an amphoteric surfactant and an ionic polymer and (c) an opacifier agent comprising an aqueous polymer dispersion prepared by emulsion polymerization of monomers consisting essentially of styrene in an aqueous solution containing a water-soluble or a water-dispersible copolymer having hydrophobic groups and carboxyl groups and a nonionic surfactant and having 0.1-0.3 microns average particle diameter. Storage stability of the lotion cannot be obtained since the thickener acting as an opacifier, has inferior compatibility with other ingredients, which gives richness sensation and nourishing effect.

Another type of opalescent lotions contains an oil component, surfactant, and water with optimized amounts that are within an extremely narrow range in order to produce opalescent appearance. Since this type of lotion does not normally contain a thickener, the watery fresh sensation is excellent upon contact with the skin, whereas the richness sensation and the nourishing effect will be sparse.

The two lotion types that are described above totally differ from each other with respect to their ingredient compositions, production methods and cosmetic properties. Therefore, it has been thought that lotions sharing common features were impossible to obtain. In fact, no lotion satisfying both good watery fresh sensation, richness sensation and nourishing effect is known. This is in particular the case of the compositions that are disclosed in JP S61 289011 application that deals with a cosmetic, obtained by incorporating a mucilaginous substance of Tremella fuciformis Berk. prepared by liquid cultivation with a cosmetic base. This is the case of the further references as well. US 2016/129119 discloses dispersions containing lipid peptide type compound useful as low molecular weight gelator, such as lipid dipeptide and lipid tripeptide, and dissolution accelerator capable of dissolving the lipid peptide type compound at lower temperature and more easily. In this reference, peptide portion is formed by repetition of at least two or more identical or different amino acids is bonded to lipid portion including C10-24 aliphatic group; dissolution accelerator having, in molecules thereof, hydrophilic portion and hydrophobic portion, the hydrophilic portion having betaine structure. In US 2009/247444, post-foaming cosmetic cleansing preparations containing detersive surfactants with saturated radicals, 2.5 to 25% by volume of pure oxygen, and xanthan gum are disclosed. US 2009/280149 deals with a cosmetic containing a higher alcohol, an oily component and a polysaccharide such as Alcaligenes polysaccharide, in an emulsified state. Alcaligenes polysaccharide is further described in US 2007/066499 as a hydrophilic emollient that can be used in an aerosol composition for cleansing the skin without rinsing with water.

Accordingly, an object of the present invention is to provide an aqueous cosmetic with non-transparent appearance that delivers watery fresh sensation upon contact with the skin and that supplies richness sensation and nourishing effect during wider application to the skin. Here, "the non-transparent appearance" includes white, cloudy, turbid, translucent or opalescent appearance; a colored appearance is also acceptable.

### Solution to Problem

An aqueous cosmetic according to one embodiment of the present invention comprises: (1) at least one nature-derived polysaccharide, the polysaccharide comprising as constituent monosaccharides, glucuronic acid and only one cyclic form of an aldohexose, said one nature-derived polysaccharide being a fungus-derived Tremella fuciformis polysaccharide; (2) a particulate copolymer obtained from at least two monomer units selected from the group consisting of a monomer having an ethylenic unsaturated group and a phenyl group, and a monomer having a (meth)acryloyl group; (3) an anionic surfactant; and (4) from 50 to 95 wt % water with respect to the weight of the aqueous cosmetic. Note that (meth)acryloyl represents acryloyl or methacryloyl. For example, (meth)acrylate comprises a (meth)acryloyl group.

An "aqueous cosmetic" according to the invention is a composition comprising from 50 to 95 wt % water, more preferably from 50 to 99 wt %.

The present inventors have found that various advantageous effects are obtained when the prescribed particulate copolymer is dispersed by the anionic surfactant in water, in coexistent with the prescribed polysaccharide.

That is, the aqueous cosmetic of the present invention is not only very excellent in watery fresh sensation upon contact with the skin, but is also provided with properties that have been hitherto incompatible i.e., richness sensation and nourishing effect during wider application to the skin. Consequently, the aqueous cosmetic is suited to users who seek luxurious sensation and users who also seek both of watery fresh sensation and moisture-retaining property by virtue of their living environments and body constitutions. The aqueous cosmetic is excellent not only in these characteristics but also in storage stability. Specifically, the phase separation (or precipitation) of the ingredients, such as the particulate copolymer, does not occur under high-temperature conditions (e.g., at 50 °C) as well as under low-temperature conditions (e.g., 4 °C) for a prolonged period.

The polysaccharide may comprise, as an additional constituent monosaccharide, at least one cyclic form of a deoxyaldohexose and/or at least one cyclic form of an aldopentose. The one cyclic form of an aldohexose is preferably selected from the group consisting of glucose and mannose. A cyclic form of a deoxyaldohexose may be fucose and/or rhamnose. A cyclic form of an aldopentose may be xylose.

The polysaccharide may for example comprise as constituent monosaccharides, the combination of i) glucuronic acid, ii) one cyclic form of an aldohexose, and iii) at least one cyclic form of a deoxyaldohexose and/or at least one of one cyclic form of an aldopentose. Glucose or mannose may be mentioned as one cyclic form of an aldohexose; fucose or rhamnose may be mentioned as one cyclic form of a deoxyaldohexose; and xylose may be mentioned as one cyclic form of an aldopentose.

Where the polysaccharide obtained from those constituent monosaccharides is combined with the above-described particulate copolymer and surfactant, it imparts especially excellent stability to the aqueous cosmetic with non-transparent appearance. The richness sensation and the nourishing effect during wider application to the skin are far more superior.

The monomer comprising an ethylenic unsaturated group and a phenyl group is preferably styrene or a substituted styrene, and the monomer comprising a (meth)acryloyl group is preferably a (meth)acrylate. In addition, the average particle size of the particulate copolymer can be from 10 to 1,000 nm. The average particle size (particle size distribution) can be measured using a dynamic light scattering method (DLS, Delsa Max CORE by Beckman coulter, Inc.) The particulate copolymer that is obtained from such monomers and having particle size is effective for making the cosmetic non-transparent; and when combined with the aforementioned polysaccharide and the surfactant, it has excellent stability. It also contributes to the watery fresh sensation upon contact with the skin and to the richness sensation and the nourishing effect during wider application to the skin.

The viscosity of the aqueous cosmetic at 25 °C is preferably from 10 to 500 mPa.s. The viscosity can be obtained by shear viscosity measurement using a rotational rheometer (Rheolab^{®} QC manufactured by Anton Pearl GmbH) at a revolution speed of 200 rpm and at 25 °C. Within such a viscosity range, the watery fresh sensation upon contact with the skin will be particularly marked.

Moreover, the aqueous cosmetic may further comprise at least one polyhydric alcohol. The polyhydric alcohol can be a mixture of an alkylene glycol and glycerol.

The particulate copolymer can be a particle styrene/acrylates copolymer and the anionic surfactant can be sodium lauryl sulfate.

In a preferred embodiment, the aqueous cosmetic comprises,
(a) *Tremella Fuciformis* polysaccharide,
(b) styrene/acrylates copolymer,
(c) sodium lauryl sulfate,
(d) butylene glycol and/or glycerol. and
(e) from 50 to 95 wt % water with respect to the weight of the aqueous cosmetic.

In this embodiment, the contents of (a), (b), (c) and (d) can be from 0.01 to 1 wt %, from 0.01 to 0.5 wt %, from 0.00025 to 0.0125 wt % and from 5 to 30 wt % respectively with respect to the weight of the aqueous cosmetic.

### Advantageous Effects of Invention

According to the present invention, there is provided an aqueous cosmetic that delivers watery fresh sensation upon contact with the skin and that supplies richness sensation and nourishing effect during wider application to the skin.

### Description of Embodiments

The preferred embodiments of the present invention will be described below; however, the present invention shall not be limited to the embodiments below in any way.

The aqueous cosmetic comprises at least one nature-derived polysaccharide (called component (1)), said polysaccharide comprising as constituent monosaccharides, glucuronic acid and one cyclic form of an aldohexose, and being a fungus-derived Tremella fuciformis polysaccharide. Component (1) may work as a thickener. As used herein, "nature-derived polysaccharide" means a compound that is taken out from a plant (plant-derived) or from a fungus (fungus-derived, for example *Basidiomycota, Ascomycota*) through extraction or the like. "Nature-derived" may also mean "bacterium-produced" i.e. a compound that is purified or processed from a substance (for example a macromolecular substance) that is produced by a bacterium.

The one cyclic form of an aldohexose is preferably a pyranose, that is, a six-membered ring composed of five carbon atoms and one oxygen atom. The one cyclic form of an aldohexose is preferably selected from the group consisting of allose, altrose, glucose, mannose, gulose, idose, galactose, and talose. Among these, glucose or mannose is preferred as the one cyclic form of an aldohexose.

The at least one nature-derived polysaccharide may comprise as an additional constituent monosaccharide, other constituent monosaccharides, insofar as said polysaccharide comprises glucuronic acid and only one cyclic form of an aldohexose as mentioned above. The one cyclic form of an aldohexose may be selected from the group consisting of glucose and mannose. Specifically, it may comprise as an additional constituent monosaccharide, at least one cyclic form of a deoxyaldohexose (e.g. fucose, fuculose, and rhamnose) and/or at least one cyclic form of an aldopentose (e.g. ribose, arabinose, xylose, and lyxose). It is preferable that at least one cyclic form of a deoxyaldohexose is fucose, or fucose and rhamnose. It is also preferable that one cyclic form of an aldopentose is xylose. According to an embodiment of the invention the at least one nature-derived polysaccharide comprises as additional constituent monosaccharides, no cyclic form of a deoxyaldohexose, and one cyclic form of an aldopentose, for example xylose.

According to a preferred embodiment of the invention, the aqueous cosmetic may comprise one nature-derived polysaccharide (A) comprising as additional constituent monosaccharides, a combination of two cyclic forms of a deoxyaldohexose, for example fucose and rhamnose. In another embodiment, one nature-derived polysaccharide (B) comprises as an additional constituent monosaccharides a combination of one cyclic form of a deoxyaldohexose, for example fucose, and one cyclic form of an aldopentose, for example xylose. According to a further embodiment of the invention, the aqueous cosmetic comprises a mixture of polysaccharide (A) and polysaccharide (B).

As used herein, the "one cyclic form of an aldohexose" means that the nature-derived polysaccharide comprises as a constituent monosaccharide only one aldohexose being in a cyclic form. This wording excludes a nature-derived polysaccharide comprising as constituent monosaccharides two aldohexoses being in a cyclic form. The wording "one cyclic form of a deoxyaldohexose" preferably refers to pyranose having a cyclic structure derived from a deoxyaldohexose, that is a six-membered ring composed of five carbon atoms and one oxygen atom. The "cyclic form of an aldopentose" refers to a cyclic pentose derived from an aldopentose, having a six-membered ring composed of five carbon atoms and one oxygen atom or a five-membered ring composed of four carbon atoms and one oxygen atom.

The at least one nature-derived polysaccharide may be a mixture of high-molecular weight polysaccharide chains and low-molecular weight polysaccharide chains. As used herein, "high-molecular weight" means an average molecular weight of 100,000 or more, and preferably 1,000,000 or more, measured by size exclusion chromatography, and the distribution of the molecular weight is preferably confined between 10⁶ and 10⁹. "Low-molecular weight" means an average molecular weight of less than 100,000, and preferably less than 1,000,000 measured by the same measurement method, and the distribution of the molecular weight is preferably confined between 10³ and 10⁷.

The aqueous cosmetic of the invention may comprise other polysaccharides, preferably a low-molecular weight polysaccharide, in addition to the polysaccharide molecule comprising as constituent monosaccharides, glucuronic acid and one cyclic form of an aldohexose. As the other polysaccharide, there may be mentioned a polysaccharide which does not comprise glucuronic acid as the constituent monosaccharide but which comprises as the constituent monosaccharides, a) one cyclic form of an aldohexose (e.g., allose, altrose, glucose, mannose, gulose, idose, galactose, and talose) and/or b) at least one cyclic form of a deoxyaldohexose (e.g., fucose, fuculose, and rhamnose). A low-molecular weight polysaccharide comprising as the constituent monosaccharides, mannose and fucose is preferable as the other polysaccharide.

It is preferred that the weight percentage of glucuronic acid is higher or equal to 20 wt % by mole, with respect to the weight of the constituent monosaccharides.

As the fungus-derived Tremella fuciformis polysaccharide, there may be mentioned Tremoist^{®} TP as trade name (INCI name: Tremella fuciformis polysaccharide), which is manufactured by Nippon Fine Chemical Co., Ltd. This at least one nature-derived polysaccharide has i) mannose as a principle chain, and ii) xylose and glucuronic acid as side chains, the mole percentage of glucuronic acid being approximately 20 wt % by mole of the mole number of the constituent monosaccharides. An average molecular weight of Tremoist^{®} TP is 1,000,000 or more.

According to the finding of the present inventors, when replacing component (1) with i) a xanthan gum having as constituent monosaccharides, glucuronic acid, glucose and mannose, with ii) hyaluronic acid comprising as constituent monosaccharides, glucuronic acid and N-acetylglucosamine, or with iii) hydroxyethyl cellulose having as the constituent monosaccharide, glucose, the transformation of the watery fresh sensation to the richness sensation and the nourishing effect does not occur.

The aqueous cosmetic comprises a particulate copolymer (component (2)) obtained from at least two monomer units selected from the group consisting of a monomer comprising an ethylenic unsaturated group and a benzene skeleton and a monomer comprising a (meth)acryloyl group, in addition to component (1). Component (2) may work as an opacifier.

The non-transparent appearance of the aqueous cosmetic is mainly caused by these particulate copolymers. The average particle size of the particulate copolymer is preferably from 10 to 1,000 nm for dispersing light. The average particle size is preferred to be from 10 to 800 nm, more preferably from 20 to 500 nm, and particularly from 50 to 300 nm. Note that the average particle size can be measured by dynamic light scattering.

As the monomer having an ethylenic unsaturated group and a benzene skeleton, which may be referred to as "monomer A", an ethylenic unsaturated group, such as a vinyl group or a vinylidene group, can be bonded directly, or bonded through a divalent group, to the benzene skeleton. Parts of hydrogen atoms of the benzene skeleton may be substituted with substituents such as a methyl group, a hydroxyl group, and a halogen atom (e.g., chlorine atom). Typical examples of monomer A are styrene and a substituted styrene. The substituents of the substituted styrene are as illustrated above. As the substituted styrene, there may be mentioned alpha-styrene, methylstyrene, hydroxystyrene, or chlorostyrene.

As the monomer comprising a (meth)acryloyl group, which may be referred to as "monomer B," a monomer such as (meth)acrylic acid, a

(meth)acrylate [i.e., (meth)acrylic acid ester] , or a (meth)acryl amide can be used. The (meth)acrylic acid esters include i) alkyl esters of (meth)acrylic acid having an alkyl group, ii) from C1 to C22, preferably from C1 to C18, and more preferably from C1 to C12, and ii) a (meth)acrylate wherein part of hydrogen atoms of said alkyl group is substituted with a hydroxyl group, an amino group, or the like [e.g., hydroxyalkyl (meth)acrylate, aminoalkyl (meth)acrylate, N-methylaminoalkyl (meth)acrylate, and N,N-dimethylaminoalkyl (meth)acrylate].

The particulate copolymer consists of at least one monomer A and at least one monomer B as at least two monomer units (i.e., repeating units); it is not impermissible to have as at least two monomer units, monomers other than those mentioned. As the monomer other than monomer A and monomer B, there may be mentioned a monomer having "an ethylenic unsaturated group but no benzene skeleton," such as ethylene or ethyl acetate. The ratio of monomer A to monomer B or where other monomer is included, the ratio of all monomers containing said monomer can be determined by the calculation by means of FOX equation or the like so that the glass transition temperature (Tg) of the particulate copolymer formed may be greater than room temperature (e.g., 25 °C).

It is necessary that such a copolymer has a particulate form. The particulate form may be obtained by bulk polymerization or solution polymerization, removal of volatiles therefrom followed by pulverization of said copolymer. However, it is preferred that the particulate copolymer is formed in the particulate form in a medium such as water by emulsion polymerization or suspension polymerization of the copolymer, from the viewpoint of ease of manufacturing and uniformity of the particle size. The emulsion polymerization or the like may contain water that is used as a medium; this water content has to be taken into account to calculate the total water amount in the aqueous cosmetic, which is prepared by mixing the emulsion polymerization, water and other ingredients. Specifically, the emulsion of the particulate copolymer obtained from monomer A and monomer B as at least two monomer units can be incorporated as such in the aqueous cosmetic. Furthermore, if an anionic surfactant is used as a surfactant in a case where the emulsion polymerization or the like is carried out, the surfactant may be used as an anionic surfactant to be contained in the aqueous cosmetic. Styrene/acrylates copolymer is preferably used as the particulate copolymer.

The aqueous cosmetic comprises an anionic surfactant (component (3)) in addition to components (1) and (2) mentioned above. Component (3) may work as a dispersant of component (2). Regarding component (3), it is sufficient that the capability of dispersing component (2) may be exerted in water.

As the anionic surfactant, there may be mentioned an alkylbenzene-based anionic surfactant such as sodium alkylbenzene sulfate; a higher alcohol-based anionic surfactant such as sodium alkyl sulfate ester or sodium alkylether sulfate ester; an alpha-olefin-based anionic surfactant such as sodium olefin sulfate; a normal paraffin-based anionic surfactant such as sodium alkyl sulfate; and a fatty acid-based anionic surfactant such as fatty acid sodium or sodium sulfonated fatty acid alpha ester. A typical example of sodium alkylbenzene sulfate is sodium dodecylbenzene sulfate; and a typical example of the sodium alkyl sulfate is sodium lauryl sulfate.

The surfactant that is used to produce the particulate copolymer suspension can be used as component (3). As a particulate copolymer suspension dispersed by an anionic surfactant, there may be mentioned ACUSOL^{®} OP 301 (INCI name: Styrene/acrylates copolymer, Sodium lauryl sulfate, water), which is manufactured by Rohm and Hass.

The aqueous cosmetic may comprise another surfactant, including a cationic surfactant or a non-ionic surfactant, insofar as it comprises the anionic surfactant. However, it is preferred that the aqueous cosmetic only comprises the anionic surfactant. In addition, the anionic surfactant may be present as one type or plural types. Sodium alkyl sulfate ester, such as sodium lauryl sulfate, is particularly preferred as the anionic surfactant. In a particular embodiment, the particulate copolymer is a styrene/acrylates copolymer and the anionic surfactant is sodium lauryl sulfate.

The aqueous cosmetic comprises from 50 to 95 wt % water with respect to the weight of the aqueous cosmetic, in addition to components (1)-(3). As used herein, "with respect to the weight of the aqueous cosmetics" also means "of the total weight of the aqueous cosmetic".

Water can be a medium to dissolve components (1) and (3) and can also be a medium to disperse component (2). Water is an essential ingredient for the watery fresh sensation upon contact with the skin. Water can be steam-distilled water derived from plants, which includes lavender water, rose water, and orange flower water, purified water, hot spring water, or deep water. The content of water is preferably from 60 to 95 wt %, more preferably from 70 to 95 wt %, and in particular from 85 to 95 wt % with respect to the weight of the aqueous cosmetic.

Since the preferable water content of the aqueous cosmetic is as described above, it is preferred that the remaining parts are composed of the other ingredients. Specifically, the content of component (1) is preferably from 0.01 to 1 wt %, more preferably from 0.02 to 0.8 wt %, and more preferably from 0.03 to 0.3 wt %. The content of component (2) is preferably from 0.01 to 0.5 wt %, more preferably from 0.05 to 0.4 wt %, and more preferably from 0.15 to 0.35 wt %. The content of component (3) is preferably from 0.00025 to 0.0125 wt %, more preferably from 0.00025 to 0.01 wt %, and more preferably from 0.00025 to 0.008 wt %.

The particulate copolymer is a raw material that is mixed with other ingredients to prepare the aqueous cosmetic. In the case that the particulate copolymer is introduced in the form of a dry powder, the weight percentage of the particulate copolymer with respect to the weight of the aqueous cosmetic may represent the weight percentage of the powder with respect to the weight of the aqueous cosmetic. Furthermore, in the case that the particulate copolymer is introduced as a raw material that is in the form of an emulsion particulate copolymer comprising copolymer particles, water and a surfactant, the weight percentage of the particulate copolymer with respect to the weight of the aqueous cosmetic may represent either the weight percentage of the dry copolymer particles with respect to the weight of the aqueous cosmetic, or the weight percentage of the emulsion particulate copolymer with respect to the weight of the aqueous cosmetic.

The viscosity of the aqueous cosmetic can be, for example, set within the range of from 10 to 500 mPa.s., which is measured by shear viscosity measurement using a rotational rheometer (Rheolab QC manufactured by Anton Pearl GmbH) at a speed revolution of 200 rpm and at 25 °C. The aqueous cosmetic will deliver the watery fresh sensation upon contact with the skin and will generate the richness sensation and the nourishing effect during wider application to the skin. If the above-described viscosity range is set, the user will be provided with satisfaction against the hitherto non-existing performance while being provided with unexpectedness because of noteworthy manifestation of the transformation. The viscosity of the aqueous cosmetic is preferably from 10 to 500 mPa.s, and more preferably from 20 to 400 mPa.s.

The aqueous cosmetic can appropriately be compounded with an oil agent, a moisturizing agent, an UV absorber, an antioxidant, a browndiscoloration preventer, a preservative, a cosmetically-active ingredient, a stabilizer, a dye, a perfume, and any other ingredient, so that a variety of effects may be imparted.

One or two or more polyhydric alcohols can be used as the moisturizing agent. As the polyhydric alcohol, there may be mentioned a glycol such as polypropylene glycol, 1,3-butylene glycol, dipropylene glycol, or polyethylene glycol; and a glycerol such as glycerol, diglycerol, or polyglycerol. Any plant oil, and any synthetic oil can be used as the oil agent. Such an oil agent can be a hydrocarbon oil, a fat, a wax, a hydrogenated oil, an ester oil, a fatty acid, a higher alcohol, a silicone oil, a fluorinated oil, and others. The content of polyhydric alcohol is preferably from 5 to 30 wt %, preferably from 5 to 25 wt %, and pre preferably from 5 to 15 wt % of weight of the aqueous cosmetic.

Production of the aqueous cosmetic can be carried out in the manner that follows, for example. Specifically, component (2) is dispersed in water with component (3) to obtain a dispersion that is added to water where component (1) is dissolved or dispersed; sufficient agitation makes said production possible. When component (2) is in powder form, component (2) may be added to water in which components (1) and (3) are dissolved or dispersed, and sufficient agitation may be made. When the aqueous cosmetic comprises the above-described additives, the addition is possible at any stage of said production process.

The aqueous cosmetic can be, for example, utilized as a lotion, a milky lotion, a serum or a cosmetic base.

### Examples

The aqueous cosmetic will be further described hereinbelow by way of the examples and comparative examples of the present invention; however, the present invention is not limited to the examples below.

Table 1 shows a list of trade names, manufacturers, and components that are used as raw materials in the examples and comparative examples. INCI means International Nomenclature of Cosmetic Ingredients.

**[Table 1]**

| **Trade Name** | **Manufacturer** | **Component** |
|---|---|---|
| Alcasealan^{®} | Hakuto Co., Ltd. | INCI: Alcaligenes polysaccharide |
| Tremoist^{®} TP | Nippon Fine Chemical Co. Ltd. | INCI: Tremella fuciformis polysaccharide |
| Bashyal^{®} Poudre | SOLIANCE | INCI: Sodium hyaluronate |
| Hyaluronate sodium HPN^{®} | Health Plan of Nevada Inc. | INCI: Sodium hyaluronate |
| Rhodicare^{®} XC | Solvay S.A. | INCI: Xanthan gum |
| Rhodicare^{®} T | Solvay S.A. | INCI: Xanthan gum |
| Natrosol^{®} 250M | | INCI: hydroxyethyl cellulose |
| Acusol^{®} OP 301 | Rhom and Hass | INCI: Styrene/acrylates copolymer, Sodium lauryl sulfate, Water |
| Syntran^{®} 5905 | Interpolymer Corporation | INCI: Styrene/acrylates copolymer, C11-15 Pareth-40, C11-15 Pareth-7, Water |
| Nikkofine^{®} LW | Nikko Chemicals Co., Ltd. | Mixture containing Polyoxyethylene Hydrogenated Castor Oil |
| Euperlan^{®} PK 771BENZ | BASF | INCI: Glycerol distearate, Sodium lauryl sulfate, Laureth-10, Benzoic acid, Cocamide MEA, Water |

Chemicals that are identified in Table 2 as opacifiers and surfactants were added to water in which chemicals that are identified in Table 2 as thickeners and moisturizing agents have been previously dissolved; sufficient agitation was carried out to obtain the cosmetics of Example 1 and Comparative Examples 1-8. The compounding proportions (by weight) are as shown in Table 2. The solid portion of Acusol^{®} OP 301 is about 40 wt %. In the product, a water content is 59-61 wt %, a styrene/acrylates copolymer amount is 38-40 wt %, sodium lauryl sulfate content amounting for the rest. The cosmetics of Example 1 and Comparative Examples 1-8 were evaluated for their viscosities at 25 °C as well as for their stabilities at 4 °C and 50 °C according to the method shown below.
(1) Viscosity at 25 °C: Shear viscosity measurement using a rotational rheometer (Rheolab^{®} QC manufactured by Anton Pearl GmbH) was done to determine viscosities at a revolution speed of 200 rpm and at 25 °C.
(2) Stabilities at 4°C and 50°C: The cosmetics of Example 1 and Comparative Examples 1-8 were accommodated in transparent containers, and after having been sealed with lids, they were stored at 4°C or 50°C for one month. After storage, they were brought to room temperature (25°C) and observed for the absence or presence of precipitates from the opacifiers and others. Those for which there was no precipitation either at 4°C or at 50°C and which were excellent in the stability were denoted "Y"; and those for which the precipitation was formed either at 4 °C or at 50 °C were denoted "N."

**[Table 2]**

| Component | Example | Comparative | Comparative | Comparative | Comparative | Comparative | Comparative | Comparative | Comparative |
|---|---|---|---|---|---|---|---|---|---|
| Trade Name or INCI name | 1 | Example 1 | Example 2 | Example 3 | Example 4 | Example 5 | Example 6 | Example 7 | Example 8 |
| Alcasealan^{®} | 0,1 | 0,1 | 0,1 | 0,1 | - | - | - | - | - |
| Tremoist^{®} TP | 0,1 | 0,1 | 0,1 | 0,1 | - | - | - | - | - |
| Bashyal^{®} Poudre | - | - | - | - | 0,2 | - | - | - | - |
| Hyaluronate sodium HPN^{®} | - | - | - | - | - | 0,2 | - | - | - |
| Rhodicare^{®} XC | - | - | - | - | - | - | 0,2 | - | - |
| Rhodicare^{®} T | - | - | - | - | - | - | - | 0,2 | - |
| Natrosol^{®} 250M | - | - | - | - | - | - | - | - | 0,2 |
| Acusol^{®} OP 301 | 0,5 | - | - | - | 0,5 | 0,5 | - | - | - |
| Syntran^{®} 5905 | - | 0,5 | - | - | - | - | - | - | - |
| Nikkofine^{®} LW | - | - | 0,5 | - | - | - | - | - | - |
| Euperlan^{®} PK 771BENZ | - | - | - | 0,5 | - | - | - | - | - |
| INCI: Water | 89,3 | 89,3 | 89,3 | 89,3 | 89,3 | 89,3 | 89,3 | 89,3 | 89,3 |
| INCI:Butylene glycol | 5 | 5 | 5 | 5 | 5 | 5 | 5 | 5 | 5 |
| INCI: Glycerin | 5 | 5 | 5 | 5 | 5 | 5 | 5 | 5 | 5 |
| Viscosity (mPas, 25 °C) | 131,2 | 101,2 | 182,8 | 180,6 | 15,56 | 105 | 33,2 | 42,3 | 15,5 |
| Stability (50°C x one month, 4°C x one month) | Y | N | N | N | N | N | N | N | N |

Chemicals that are mentioned in Table 3 as being opacifiers and surfactants were added to water in which chemicals that are mentioned in Table 3 as being thickeners and moisturizing agents have been previously dissolved or dispersed, and sufficient agitation was carried out to obtain the cosmetics of Examples 1-4 and Comparative Examples A and B. The compounding proportions (by weight) are as shown in Table 3. The cosmetics of Examples 1-4 and Comparative Examples A and B were measured for their viscosities at 25 °C according to the above-described method and were evaluated for the richness sensation and the nourishing effect, as well as for the watery fresh sensation according to the methods shown below.
(3) Richness sensation and nourishing effect: Evaluation was made, in a single use test on the entire face, by a 10 in-house cosmetic-specialized-person panel.
(4) Watery fresh sensation: Evaluation was made, in a single use test on the entire face, by a 10 in-house cosmetic-specialized-person panel.

The richness sensation, the nourishing effect, and the watery fresh sensation were evaluated in terms of: "A" - markedly noted; "B" - noted; "C" - not sufficiently noted; "D" - not noted.

**[Table 3]**

| Trade Name or INCI NAME | Example 1 | Example 2 | Example 3 | Example 4 | Comparative | Comparative |
|---|---|---|---|---|---|---|
| | | | | | Example A | Example B |
| Alcasealan^{®} | 0,1 | - | - | - | 0 | 0,1 |
| Tremoist^{®}TP | 0,1 | 0,05 | 0,1 | 0,2 | 0 | 0,1 |
| Acusol^{®} OP 301 | 0,5 | 0,5 | 0,5 | 0,5 | 0,5 | 0,5 |
| Water | 89,3 | 89,3 | 89,3 | 89,3 | 89,3 | 49,3 |
| 1,3-Butylene glycol | 5 | 5 | 5 | 5 | 5 | 25 |
| Glycerol | 5 | 5 | 5 | 5 | 5 | 25 |
| Viscosity (mPas, 25 °C) | 131,2 | 14,73 | 30,1 | 74,33 | 5,8 | 236,6 |
| Richness sensation and Nourishing effect | A | B | B | A | D | A |
| Watery fresh sensation | A | A | A | B | A | D |

## Claims

1. An aqueous cosmetic comprising:
at least one nature-derived polysaccharide comprising, as constituent monosaccharides, glucuronic acid and only one cyclic form of an aldohexose, said one nature-derived polysaccharide being a fungus-derived *Tremella fuciformis* polysaccharide,
a particulate copolymer obtained from at least two monomer units selected from the group consisting of a monomer comprising an ethylenic unsaturated group and a phenyl group, and a monomer comprising a (meth)acryloyl group;
an anionic surfactant; and
from 50 to 95 wt % water with respect to the weight of the aqueous cosmetic.

2. The aqueous cosmetic according to claim 1, wherein the polysaccharide comprises, as an additional constituent monosaccharide, at least one cyclic form of a deoxyaldohexose and/or at least one cyclic form of an aldopentose.

3. The aqueous cosmetic according to claim 1 or 2, wherein the only one cyclic form of an aldohexose is selected from the group consisting of glucose and mannose.

4. The aqueous cosmetic according to claim 2 or 3, wherein a cyclic form of a deoxyaldohexose is fucose and/or rhamnose.

5. The aqueous cosmetic according to any one of claims 1-4, wherein the monomer comprising an ethylenic unsaturated group and a phenyl group is styrene or a substituted styrene, and the monomer comprising a (meth)acryloyl group is a (meth)acrylate.

6. The aqueous cosmetic according to any one of claims 1-5, wherein an average particle size of the particulate copolymer is from 10 nm to 1,000 nm.

7. The aqueous cosmetic according to any one of claims 1-6, having a viscosity of from 10 to 500 mPa.s at 25 °C.

8. The aqueous cosmetic according to any one of claims 1-7, further comprising at least one polyhydric alcohol.

9. The aqueous cosmetic according to claim 8, wherein the polyhydric alcohol is a mixture of an alkylene glycol and glycerol.

10. The aqueous cosmetic according to any one of claims 1-9, wherein the particulate copolymer is a styrene/acrylates copolymer and the anionic surfactant is sodium lauryl sulfate.

11. The aqueous cosmetic according to any one of claims 1-10, wherein the aqueous cosmetic comprises,
(a) *Tremella Fuciformis* polysaccharide,
(b) styrene/acrylates copolymer,
(c) sodium lauryl sulfate,
(d) butylene glycol and/or glycerol. and
(e) from 50 to 95 wt % water with respect to the weight of the aqueous cosmetic.

12. The aqueous cosmetic according to claims 11, wherein the contents of (a), (b), (c) and (d) are from 0.01 to 1 wt %, from 0.01 to 0.5 wt %, from 0.00025 to 0.0125 wt % and from 5 to 30 wt % respectively with respect to the weight of the aqueous cosmetic.

13. The aqueous cosmetic according to claim 2 or 3, wherein a cyclic form of an aldopentose is xylose.

## Patentansprüche

1. Wässriges Kosmetikum, umfassend:
zumindest ein aus der Natur abgeleitetes Polysaccharid, umfassend als Monosaccharid-Bestandteil Glucuronsäure und nur eine zyklische Form einer Aldohexose, wobei das aus der Natur abgeleitete Polysaccharid ein von einem Pilz abgeleitetes Polysaccharid von *Tremella fuciformis* ist,
ein partikelförmiges Copolymer, das aus zumindest zwei Monomereinheiten erhalten wird, die ausgewählt sind aus der Gruppe bestehend aus einem Monomer umfassend eine ethylenisch ungesättigte Gruppe und ein Phenylgruppe, und einem Monomer umfassend eine (Meth)acryloyl-Gruppe,
einen anionischen oberflächenaktiven Stoff, und
von 50 bis 95 Gew.-% Wasser bezogen auf das Gewicht des wässrigen Kosmetikums.

2. Wässriges Kosmetikum nach Anspruch 1, wobei das Polysaccharid als zusätzlichen Monosaccharid-Bestandteil zumindest eine zyklische Form einer Deoxyaldohexose und/oder zumindest eine zyklische Form einer Aldopentose umfasst.

3. Wässriges Kosmetikum nach Anspruch 1 oder 2, wobei die nur eine zyklische Form einer Aldohexose aus der Gruppe ausgewählt ist, die aus Glucose und Mannose besteht.

4. Wässriges Kosmetikum nach Anspruch 2 oder 3, wobei eine zyklische Form einer Deoxyaldohexose Fucose und/oder Rhamnose ist.

5. Wässriges Kosmetikum nach einem der Ansprüche 1-4, wobei das Monomer umfassend eine ethylenisch ungesättigte Gruppe und eine Phenylgruppe Styrol oder ein substituiertes Styrol ist, und das Monomer umfassend eine (Meth)acryloyl-Gruppe ein (Meth)acrylat ist.

6. Wässriges Kosmetikum nach einem der Ansprüche 1-5, wobei eine mittlere Partikelgröße des partikelförmigen Copolymers von 10 nm bis 1.000 nm beträgt.

7. Wässriges Kosmetikum nach einem der Ansprüche 1-6, das eine Viskosität von 10 bis 500 MPa.S bei 25 ºC aufweist.

8. Wässriges Kosmetikum nach einem der Ansprüche 1-7, ferner umfassend zumindest einen mehrwertigen Alkohol.

9. Wässriges Kosmetikum nach Anspruch 8, wobei der mehrwertige Alkohol ein Gemisch eines Alkylenglycols mit Glycerin ist.

10. Wässriges Kosmetikum nach einem der Ansprüche 1-9, wobei das partikelförmige Copolymer ein Styrol-/Acrylat-Copolymer ist und der anionische oberflächenaktive Stoff Natriumlaurylsulfat ist.

11. Wässriges Kosmetikum nach einem der Ansprüche 1-10, wobei das wässrige Kosmetikum umfasst,
(a) Polysaccharid von *Tremella Fuciformis*,
(b) Styrol-/Acrylat-Copolymer,
(c) Natriumlaurylsulfat,
(d) Butylenglycol und/oder Glycerin, und
(e) von 50 bis 95 Gew.-% Wasser bezogen auf das Gewicht des wässrigen Kosmetikums.

12. Wässriges Kosmetikum nach den Ansprüchen 11, wobei der Gehalt an (a), (b), (c) und (d) jeweils bezogen auf das Gewicht des wässrigen Kosmetikums von 0,01 bis 1 Gew.-%, von 0,01 bis 0,5 Gew.-%, von 0,00025 bis 0,0125 Gew.-% und von 5 bis 30 Gew.-% beträgt.

13. Wässriges Kosmetikum nach Anspruch 2 oder 3, wobei eine zyklische Form einer Aldopentose Xylose ist.

## Revendications

1. Produit cosmétique aqueux, comprenant :
au moins un polysaccharide dérivé de la nature, comprenant, en tant que monosaccharides constitutifs, de l'acide glucuronique et une seule forme cyclique d'un aldohexose, ledit polysaccharide dérivé de la nature étant un polysaccharide dérivé d'un champignon *Tremella fuciformis*,
un copolymère particulaire obtenu à partir d'au moins deux motifs monomères choisis dans le groupe consistant en un monomère comprenant un groupe insaturé éthylénique et un groupe phényle, et un monomère comprenant un groupe (méth)acryloyle ;
un tensioactif anionique ; et
de 50 à 95 % en poids d'eau par rapport au poids du produit cosmétique aqueux.

2. Produit cosmétique aqueux selon la revendication 1, dans lequel le polysaccharide comprend, en tant que monosaccharide constitutif additionnel, au moins une forme cyclique d'un désoxyaldohexose et/ou au moins une forme cyclique d'un aldopentose.

3. Produit cosmétique aqueux selon la revendication 1 ou 2, dans lequel la seule forme cyclique d'un aldohexose est choisie dans le groupe consistant en glucose et mannose.

4. Produit cosmétique aqueux selon la revendication 2 ou 3, dans lequel une forme cyclique d'un désoxyaldohexose est le fucose et/ou le rhamnose.

5. Produit cosmétique aqueux selon l'une quelconque des revendications 1 à 4, dans lequel le monomère comprenant un groupe insaturé éthylénique et un groupe phényle est le styrène ou un styrène substitué, et le monomère comprenant un groupe (méth)acryloyle est un (méth)acrylate.

6. Produit cosmétique aqueux selon l'une quelconque des revendications 1 à 5, dans lequel une taille moyenne de particules du copolymère particulaire va de 10 nm à 1000 nm.

7. Produit cosmétique aqueux selon l'une quelconque des revendications 1 à 6, ayant une viscosité allant de 10 à 500 mPa.s à 25°C.

8. Produit cosmétique aqueux selon l'une quelconque des revendications 1 à 7, comprenant en outre au moins un alcool polyhydrique.

9. Produit cosmétique aqueux selon la revendication 8, dans lequel l'alcool polyhydrique est un mélange d'un alkylène glycol et d'un glycérol.

10. Produit cosmétique aqueux selon l'une quelconque des revendications 1 à 9, dans lequel le copolymère particulaire est un copolymère de styrène-acrylates et le tensioactif anionique est le sodium laurylsulfate.

11. Produit cosmétique aqueux selon l'une quelconque des revendications 1 à 10, ledit produit cosmétique aqueux comprenant,
(a) un polysaccharide de *Tremella Fuciformis,*
(b) un copolymère de styrène/acrylates,
(c) du sodium laurylsulfate,
(d) du butylène glycol et/ou du glycérol, et
(e) de 50 à 95 % en poids d'eau par rapport au poids du produit cosmétique aqueux.

12. Produit cosmétique aqueux selon la revendication 11, dans lequel les teneurs de (a), (b), (c) et (d) vont de 0,01 à 1 % en poids, de 0,01 à 0,5 % en poids, de 0,00025 à 0,0125 % en poids et de 5 à 30 % en poids, respectivement, par rapport au poids du produit cosmétique aqueux.

13. Produit cosmétique aqueux selon la revendication 2 ou 3, dans lequel une forme cyclique d'un aldopentose est le xylose.
